(19)

Europäisches
Patentamt

European
Patent Office

Office européen
des brevets

(11)  **EP 4 454 683 A1**

(12)  **EUROPEAN PATENT APPLICATION**

(43) Date of publication:
**30.10.2024 Bulletin 2024/44**

(21) Application number: **23187870.3**

(22) Date of filing: **26.07.2023**

(51) International Patent Classification (IPC):
***A61M 5/315*** (2006.01)

(52) Cooperative Patent Classification (CPC):
**A61M 5/31513**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.04.2023 EP 23315131**

(71) Applicant: **Becton Dickinson France
38800 Le Pont-de-Claix (FR)**

(72) Inventors:
• **FLIPPE, Marc**
  **38640 CLAIX (FR)**
• **EUVRARD, Nicolas**
  **London, SW17 0JF (GB)**
• **DELEUIL, Nicolas**
  **38100 Grenoble (FR)**
• **POUGET, Gaëlle**
  **38100 GRENOBLE (FR)**
• **LEVERD, William**
  **38430 Moirans (FR)**

(74) Representative: **Germain Maureau**
  **12, rue Boileau**
  **69006 Lyon (FR)**

(54)  **PFAS-FREE SYRINGE PLUNGER STOPPER WITH CURVED INTER-RIB REGION**

(57)  Provided herein is a stopper free or substantially free of PFAS and adapted for attachment with a plunger rod for use within a syringe barrel. The stopper includes a main body portion defining a proximal end and a distal end, with the proximal end configured to mate with a distal end of the plunger rod. The stopper also includes a plurality of ribs each extending from an outer surface of the main body portion and around an outer circumference of the main body portion, the plurality of ribs including at least a first rib and a second rib spaced from the first rib. The stopper also includes a curved inter-rib region positioned between each pair of adjacent ribs of the plurality of ribs where the outer surface of the main body portion is curved radially inward.

EP 4 454 683 A1

**Description**

**BACKGROUND OF THE INVENTION**

Field of the Invention

The present disclosure relates generally to a plunger stopper for use with a syringe and, more particularly, to a PFAS-free plunger stopper with a curved inter-rib region.

Description of Related Art

Medical injection devices, such as syringes, are used in a variety of environments for administering liquids (e.g., medications or drugs) to a patient. Many syringes are provided as prefilled syringes, which provide the convenience of rapidly delivering the liquid therein to a patient without the need to first aspirate the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper or plunger stopper is typically made of elastomeric material and is adapted to ensure the container closure integrity of a syringe when the stopper is inserted into the syringe.

Existing stopper designs include a stopper body having a tail portion disposed at its proximal end adapted for attachment to the distal end of the plunger rod, and a head portion disposed at its distal end adapted to interfit with the barrel of the syringe. The head portion of the stopper may include a plurality of annular, outwardly protruding ribs formed on an external cylindrical wall thereof, with the ribs forming a seal with the syringe barrel to ensure the container closure integrity of the syringe. A series of two or three ribs, for example, may be spaced apart longitudinally along the stopper main body, with each pair of adjacent ribs separated by an inter-rib region provided therebetween. The inter-rib region is generally formed to present a cylindrical outer surface on the stopper between the ribs (i.e., a "straight" inter-rib region) that is inset radially inward from the ribs.

While existing stoppers with a structure as described above are effective at maintaining closure integrity in the syringe, it is recognized that such stoppers may can present issues during a syringe stoppering process, such as during insertion of the stopper into the syringe barrel during a vent tube stoppering process. That is, the design of the stopper - and specifically of the straight inter-rib region - can lead to increased friction between the stopper and the syringe barrel that makes insertion of the stopper more difficult. This increase in friction may be especially true for a stopper that is formed to be free of per- and poly- fluoroalkyl substances (PFAS).

Accordingly, a need exists in the art for a plunger stopper for use with a syringe, where the stopper is constructed to reduce the amount of friction present between the stopper and the syringe barrel during a stopper insertion process.

**SUMMARY OF THE INVENTION**

Provided herein is a stopper free or substantially free of per- and poly- fluoroalkyl substances (PFAS) and adapted for attachment with a plunger rod for use within a syringe barrel. The stopper includes a main body portion defining a proximal end and a distal end, with the proximal end configured to mate with a distal end of the plunger rod to secure the stopper to the plunger rod. The stopper also includes a plurality of ribs each extending from an outer surface of the main body portion and around an outer circumference of the main body portion, the plurality of ribs including at least a first rib and a second rib spaced from the first rib. The stopper also includes an inter-rib region positioned between each pair of adjacent ribs of the plurality of ribs, characterized in that the inter-rib region comprises a curved inter-rib region where the outer surface of the main body portion is curved radially inward.

In certain configurations, the stopper is free of PFAS.

In certain configurations, the stopper contains 200 parts-per-million or less of PFAS, and preferably 50 parts-per-million or less of PFAS

In certain configurations, the inter-rib region includes a first abutment region positioned adjacent the first rib, a second abutment region positioned adjacent the second rib, and a central region positioned between the first and second abutment regions, wherein each of the first abutment region and the second abutment region is generally flat, and wherein the central region curves radially inward from the first and second abutment regions.

In certain configurations, the first and second abutment regions provide a maximum inter-rib region outer diameter, $D_{max\_inter-rib}$, and wherein a center point of the central region provides a minimum inter-rib region outer diameter, $D_{min\_inter-rib}$.

In certain configurations, wherein the first and second ribs have an outer diameter, $D_{outer\_ribs}$ and wherein a ratio of $\frac{D_{outer\_ribs}}{D_{min\_inter-rib}}$ is between a value of 1.03 and 1.30.

In certain configurations, wherein a radius of curvature of the central region is between 30mm and 600 mm.

In certain configurations, each of the first rib and the second rib comprises a radially outward-directed contact surface, and wherein a width of the contact surface is between 0.2 and 10 mm and preferably between 0.2 mm and 1.5 mm.

In certain configurations, the stopper also includes a coating applied to at least a radially outward-directed contact surface of the first rib and the second rib, to reduce friction between the first and second ribs and the syringe barrel.

In certain configurations, the stopper includes a third rib spaced from the second rib, the third rib extending from the outer surface of the main body portion and around the outer circumference of the main body portion, with a second inter-rib region positioned between the second rib and the third rib, and with the second inter-rib region comprising a curved inter-rib region where the outer surface of the main body portion is curved radially inward.

Also provided herein is a syringe including a syringe barrel having a barrel proximal end and a barrel distal end and defining a chamber configured for containing a fluid therein, and a plunger assembly axially movable within the chamber of the syringe barrel between a retracted position and an advanced position, with the plunger assembly including a plunger having a plunger proximal end and a plunger distal end and a stopper secured to the plunger distal end, the stopper being free or substantially free of per- and poly- fluoroalkyl substances (PFAS). The stopper includes a main body portion defining a proximal end and a distal end, with the proximal end configured to mate with a distal end of the plunger rod to secure the stopper to the plunger rod. The stopper also includes a plurality of ribs each extending from an outer surface of the main body portion and around an outer circumference of the main body portion, the plurality of ribs including at least a first rib and a second rib spaced from the first rib. The stopper also includes an inter-rib region positioned between each pair of adjacent ribs of the plurality of ribs, characterized in that the inter-rib region comprises a curved inter-rib region where the outer surface of the main body portion is curved radially inward.

In certain configurations, the inter-rib region includes a first abutment region positioned adjacent the first rib, a second abutment region positioned adjacent the second rib, and a central region positioned between the first and second abutment regions, wherein each of the first abutment region and the second abutment region is generally flat, and wherein the central region curves radially inward from the first and second abutment regions.

In certain configurations, the stopper contains 0 to 200 parts-per-million of PFAS, and preferably 0 to 50 parts-per-million of PFAS.

In certain configurations, an interference between the first and second ribs and the syringe barrel is in the range of 1.5 to 20%, as determined by: Interference = (($D_{outer\_ribs}$ /$ID_{barrel}$) - 1) x 100, wherein $D_{outer\_ribs}$ is an outer diameter of the first and second ribs and $ID_{barrel}$ is an inner diameter of the syringe barrel.

**BRIEF DESCRIPTION OF THE DRAWINGS**

FIG. 1 is a perspective view of a syringe, with which embodiments of the disclosure may be implemented;

FIG. 2 is an exploded view of the syringe of FIG. 1;

FIG. 3 is a side view of a stopper included in the syringe of FIG. 1, according to a non-limiting embodiment described herein;

FIG. 4 is a side cross-sectional view of the stopper of FIG. 3 taken along line 4-4; and

FIG. 5 is a side cross-sectional view of a stopper, according to another non-limiting embodiment described herein.

**DESCRIPTION OF THE INVENTION**

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position. Thus, with a syringe for example, the distal end is the end thereof that includes a needle (or luer connection), while the proximal end is where the user engages the plunger (i.e., the plunger thumb press). Similarly, in this application, the terms "in the distal direction" and "distally" mean in the direction toward the distal tip of the syringe, and the terms "in the proximal direction" and "proximally" mean in the direction opposite the direction of the distal tip of the syringe (i.e., in the direction of the plunger thumb press).

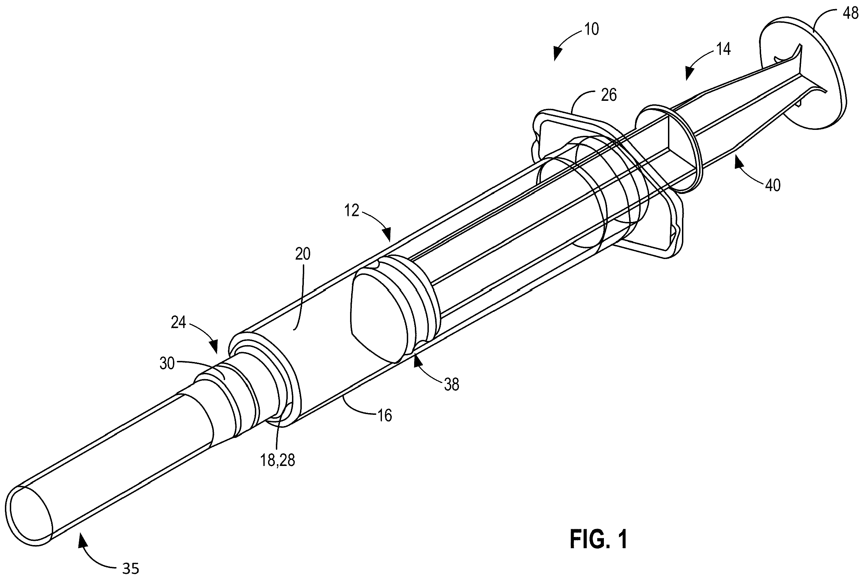

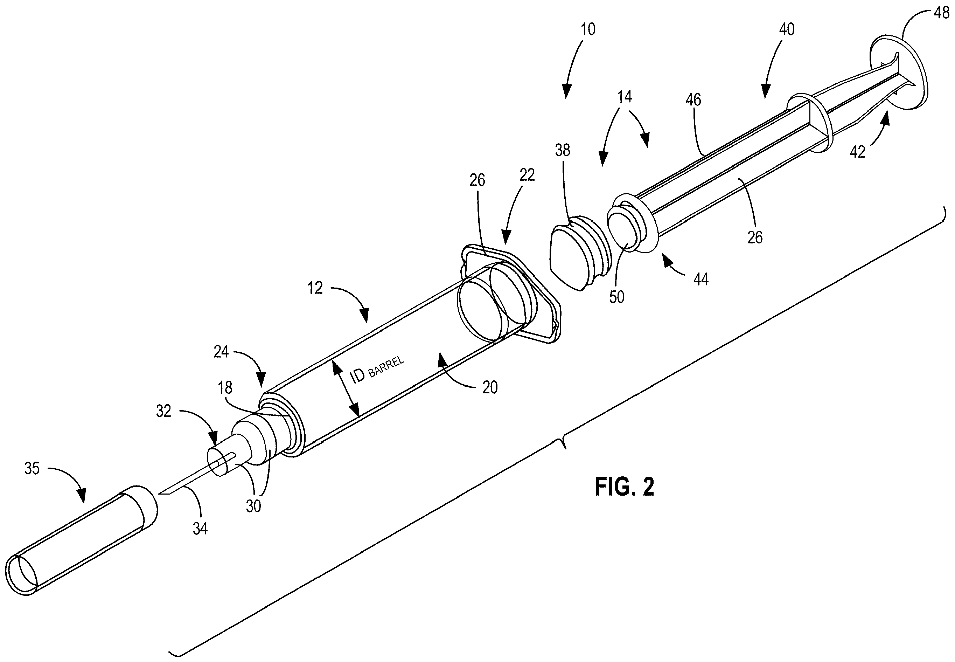

Referring to FIGS. 1 and 2, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, such as by being glued or otherwise secured to the hub portion 30.

According to some aspects of the disclosure, syringe 10 may further include a cover 35 that couples to the hub portion 30 of syringe barrel 12 to protect the needle 34. The cover 35 includes a closed distal end and an opened proximal end, with the opened proximal end enabling positioning of the cover onto hub portion 30 and over needle 34. While not shown in FIGS. 1 and 2, it is recognized that the cover 35 may comprise an outer casing made of a rigid material and an inner casing made of a soft material, such that the needle 34 may be safely housed within (or engage with) the cover 35 when the cover 35 is mounted on the hub portion 30 of the syringe 10.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. In some embodiments, an extension member 50 (e.g., threaded cylindrical member) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper 38, with the protrusion and receptacle engaging via threading, for example.

The stopper 38 of plunger assembly 14 is positioned at the plunger distal end 44 so as to be movable along with the plunger 36 within the chamber 20 of syringe barrel 12. The stopper 38 may be made from a material that is different from the material of the plunger 36 and that is capable of forming a tight seal with the syringe barrel 12 as it is advanced therethrough. In some embodiments, the stopper 38 is formed of an elastomeric material such as butyl, styrene butadiene, isoprene, as non-limiting examples. In other embodiments, the stopper 38 may instead be formed of a polymeric material, such as cross-linked or thermoplastic elastomers, as non-limiting examples.

According to aspects of the disclosure, whether the stopper 38 is formed of an elastomeric material or a polymeric material, the stopper is formed to be free or substantially free of per- and poly- fluoroalkyl substances (PFAS) - generally, a "PFAS-free" material - that might leach into the medicament or other solution stored in pre-filled syringe 10. As defined herein, the stopper 38 being "free or substantially free of PFAS" or "PFAS-free" is understood to refer to materials that are entirely free of PFAS and/or materials that may contain trace amounts of PFAS therein. In an exemplary embodiment, the stopper 38 is considered to be substantially PFAS-free if it contains 200 parts-per-million (PPM) or less of PFAS therein (i.e., no more than 200 PPM), and preferably 50 PPM or less of PFAS therein (i.e., no more than 50 PPM). In general, it is to be understood that the material from which stopper 38 is composed is to be compliant with the rules and regulations set forth by the European Union regarding limitations surrounding PFAS, as determined by the European Chemical Agency (ECHA), with proposed regulations of the ECHA being described in the ECHA Annex XV Restriction Report - Proposal for a Restriction - SUBSTANCE NAME(S): Per- and polyfluoroalkyl substances (PFASs), Version number 2, dated March 22, 2023. Thus, it is envisioned that the stopper 38 may be considered to still be "substantially PFAS-free" if it contains traces of PFAS higher than the 200 PPM indicated above (e.g., 300 or 400 PPM, as non-limiting examples), if such values are found to be acceptable/compliant with finalized guidelines or regulations set forth by the ECHA.

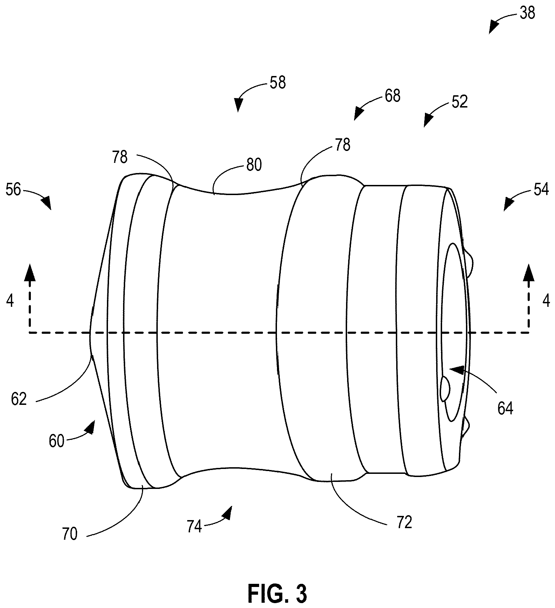

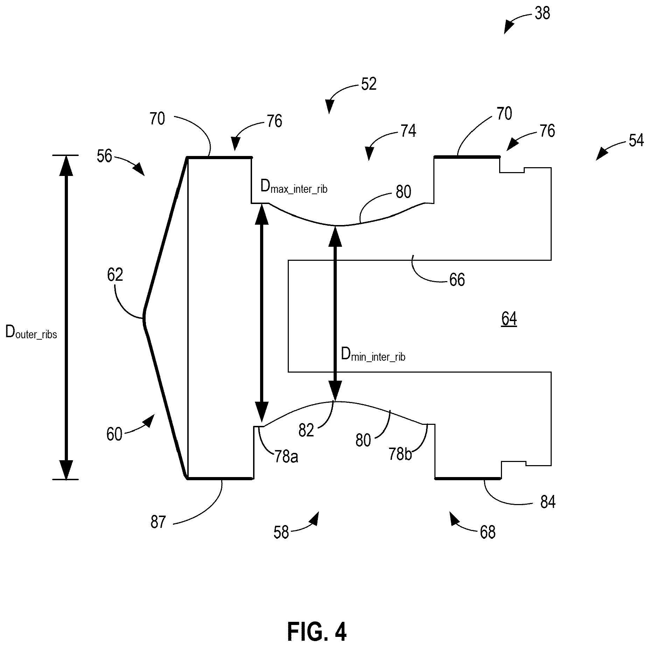

Referring now to FIGS. 3 and 4, and with continued reference to FIGS. 1 and 2, the structure of stopper 38 is shown in greater detail, according to an aspect or embodiment of the disclosure. The stopper 38 is defined by a main body 52 that includes an open proximal end 54 that engages with the distal end of plunger 36 and a closed distal end 56 configured to engage with the barrel 12 of syringe 10. The closed distal end 56 of the main body 52 includes a generally cylindrical portion 58 and a roof portion 60 that extends distally from cylindrical portion 58. In one embodiment, the roof portion 60 is configured as a conical portion that extends distally from cylindrical portion 58 to a tip 62 to provide the closed distal end 56. However, it is recognized that roof portion 60 may have other suitable shapes, such as being formed as a flat surface or domed surface, as other non-limiting examples.

As shown in FIG. 4, the main body 52 of the stopper 38 is substantially hollow and is sized and configured to receive the extension member 50 of plunger 36 therein. The main body 52 of the stopper 38 defines an inner cavity 64 that is generally defined between a majority of the open proximal end 54 and the closed distal end 56 of the main body 52. In some embodiments, and as previously indicated, the inner cavity 64 includes a threaded inner surface 66 that is configured to receive and mate with a threaded extension member 50 of plunger 36. In other embodiments, the inner cavity 64 may define an inner contoured surface having a notch therein that is configured to receive a flanged extension member of plunger 36, with the flanged extension member engaging the notch in order to retain the extension member 50 within the inner cavity 64.

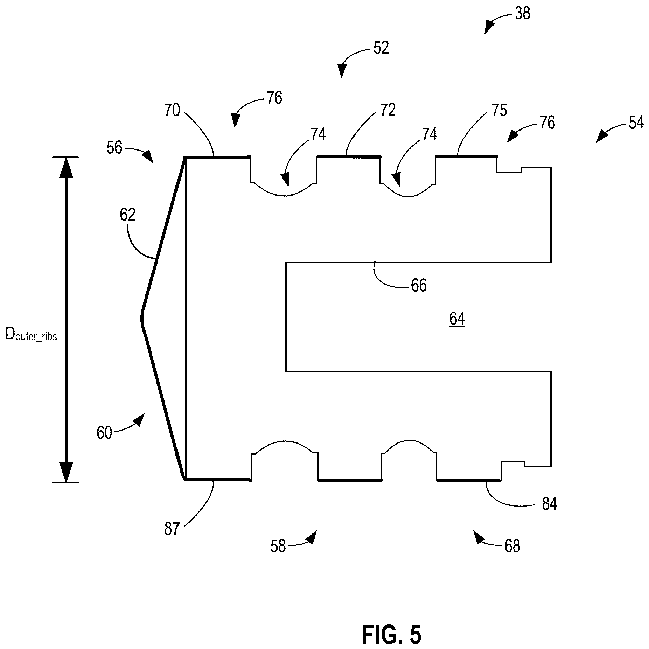

Referring still to FIGS. 3 and 4, the outer surface 68 of the cylindrical portion 58 of the main body 52 includes a plurality of ribs 70, 72 formed thereon that extend circumferentially around the entire outer surface 68 of the cylindrical portion 58. In the illustrated embodiment, the plurality of ribs 70, 72 includes a first rib 70 positioned toward the proximal end 54 of the main body 52 and a second rib 72 positioned toward the distal end 56 of the main body 52 - with the first rib 70 spaced apart longitudinally from the second rib 72 on the cylindrical portion 58 to form an inter-rib region 74 therebetween. While the stopper 38 is shown in FIGS. 3 and 4 as including only two (2) ribs 70, 72 formed thereon (i.e., first rib 70 and second rib 72), it is recognized that stopper 38 may be constructed to include a greater number of ribs formed thereon, such as three (3) ribs thereon, with a third rib 75 illustrated in FIG. 5, with an inter-rib region 74 provided between each adjacent pair of ribs.

According to embodiments of the disclosure, the structure of each of first rib 70 and second rib 72 may be controlled to achieve a desired interaction between the stopper 38 and the barrel 12. According to one aspect of ribs 70, 72, the ribs 70, 72 extend radially outward a desired distance from the inter-rib region 74 of cylindrical portion 58 (i.e., a rib thickness) to act as bearing points against the inner surface of the syringe barrel 12. In some embodiments, an outer diameter of the ribs, $D_{outer\_ribs}$, is controlled based on an inner diameter of barrel 12, $ID_{barrel}$, and to provide a desired amount of interference between the stopper 38 and the barrel 12. As one example, the rib thickness and the outer diameter of the ribs, $D_{outer\_ribs}$, is such that an interference between the stopper 38 and the syringe barrel 12 is in the range of 1.5 to 20%, as determined by: Interference = $(D_{outer\_ribs}/ID_{barrel}) - 1$) x100. According to another aspect of ribs 70, 72, a width of each of the first rib 70 and the second rib 72 that makes contact with the syringe barrel 12 (i.e., a radially outward-directed contact surface 76 of ribs 70, 72) is between 0.2 and 10 mm, and preferably between 0.2 and 1.5 mm. According to still another aspect of ribs 70, 72, each of first rib 70 and second rib 72 may include a grooving or surface texturing formed thereon, such as described in US Patent 10,751,473, which is incorporated herein by reference. The grooving or surface texturing may be formed on the radially outward-directed contact surface 76 of the ribs 70, 72 to locally decrease the contact surface thereof when compressed and locally increase the contact pressure thereof.

According to aspects of the disclosure, the inter-rib region 74 positioned between the first rib 70 and the second rib 72 (and between any other adjacent pair of ribs, if there are three or more ribs) is formed to provide a curved outer surface 68 on a section/region of the cylindrical portion 58 of the main body 52. The inter-rib region 74 includes an abutment region 78 on each end thereof adjacent the first rib 70 and second rib 72, i.e., a first abutment region 78a and a second abutment region 78b, with each of abutment regions 78 presenting a generally flat surface. A central region 80 is positioned between abutment regions 78 that curves radially inward from the abutment regions 78, such that a maximum outer diameter of inter-rib region 74, $D_{max\_inter-rib}$, is provided at abutment regions 78 and a minimum outer diameter of inter-rib region 74, $D_{min\_inter-rib}$, is provided along the central region 80 (i.e., at a longitudinal mid-point 82 of central region 80). In some embodiments, a radius of curvature of the curved central region 80 is between 30mm and 600 mm, with the curved central region 80 thus configured such that a ratio of the minimum outer diameter of inter-rib region 74, $D_{min\_inter-rib}$, to the outer diameter of the ribs, $D_{outer\_ribs}$ -- i.e., $\dfrac{D_{outer\_ribs}}{D_{min\_inter-rib}}$ -- is between a value of 1.03 and 1.30.

According to aspects of the disclosure, the stopper 38 may further include an outer surface or coating 84 applied to portions thereof, such as on a sealant or gliding surface thereof - i.e., on the contact surface 76 of first rib 70 and second rib 72 - and/or on roof portion 60. The coating 84 may be configured to reduce friction between the stopper 38 and barrel 12 when the stopper 38 is moved within the syringe 10. The coating 84 is formed of a PFAS-free material (or material that is substantially PFAS-free) that might leach into the medicament or other solution stored in pre-filled syringe 10. As described above, according to aspects of the disclosure, the coating 84 is considered to be PFAS-free or substantially PFAS-free if it contains between 0 and 200 parts-per-million (PPM) of PFAS therein and/or is otherwise compliant with guidelines or regulations set forth by the ECHA regarding acceptable amounts of PFAS.

According to aspects of the disclosure, configuring of the inter-rib region 74 as a radially inwardly curved surface reduces an amount of friction present when inserting the stopper 38 into the barrel 12 - i.e., during a stoppering process, such as vent tube stoppering. That is, inclusion of the curved inter-rib region 74 on stopper 38 lowers a contact pressure between the stopper 38 and barrel 12, specifically in the inter-rib region 74, so as to reduce the friction therebetween. In some embodiments, inclusion of the curved inter-rib region 74 on stopper 38 reduces friction between the stopper 38 and barrel 12 by 10-50% (or higher) as compared to a stopper 38 with a straight/linear inter-rib region 74, depending on the stopper sizing rules applied. In some embodiments, stoppering may be further improved (i.e., gliding optimized and friction reduced) by addition of the gliding coating 84 on ribs 70, 72. Inclusion of the gliding coating 84 on ribs 70, 72 may also minimize the need for use of a separate lubricant on stopper 38, with use of such a lubricant being eliminated or greatly reduced (e.g., $\leq 0.02$ mg /cm$^2$), when inserting the stopper 38 into syringe 10 via a vent tube stopper process.

Beneficially, embodiments of the invention thus are directed to a stopper having spaced apart first and second ribs formed thereon that extend from an outer surface of a main body portion of the stopper and extend around an outer circumference of the main body portion. An inter-rib region of the outer surface between the first and second ribs is configured as a curved inter-rib region where the outer surface of the main body portion is curved radially inward. The curved inter-rib region functions to reduce an amount of friction present when inserting the stopper into a syringe barrel, such as during a vent tube stoppering process, as the curved inter-rib region lowers a contact pressure between the stopper and barrel so as to reduce the friction therebetween. For stoppers having a PFAS-free construction/composition in particular, the curved inter-rib region is thus beneficial in enabling a stoppering process.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

**Claims**

1. A stopper free or substantially free of per- and poly- fluoroalkyl substances (PFAS) and adapted for attachment with a plunger rod for use within a syringe barrel, the stopper comprising:

   a main body portion defining a proximal end and a distal end, the proximal end configured to mate with a distal end of the plunger rod, to secure the stopper to the plunger rod;
   a plurality of ribs each extending from an outer surface of the main body portion and around an outer circumference of the main body portion, the plurality of ribs including at least a first rib and a second rib spaced from the first rib and
   an inter-rib region positioned between each pair of adjacent ribs of the plurality of ribs, **characterized in that** the inter-rib region comprises a curved inter-rib region where the outer surface of the main body portion is curved radially inward.

2. The stopper of claim 1, wherein the stopper is free of PFAS.

3. The stopper of claim 1, wherein the stopper contains 200 parts-per-million or less of PFAS, and preferably 50 parts-per-million or less of PFAS.

4. The stopper of any of claims 1-3, wherein the inter-rib region comprises:

   a first abutment region positioned adjacent the first rib;
   a second abutment region positioned adjacent the second rib; and
   a central region positioned between the first and second abutment regions;
   wherein each of the first abutment region and the second abutment region is generally flat, and wherein the central region curves radially inward from the first and second abutment regions.

5. The stopper of claim 4, wherein the first and second abutment regions provide a maximum inter-rib region outer diameter, $D_{max\_inter-rib}$, and wherein a center point of the central region provides a minimum inter-rib region outer diameter, $D_{min\_inter-rib}$.

6. The stopper of claim 5, wherein the first and second ribs have an outer diameter, $D_{outer\_ribs}$, and wherein a ratio of

$\dfrac{D_{outer\_ribs}}{D_{min\_inter-rib}}$ is between a value of 1.03 and 1.30.

7. The stopper of any of claims 4-6, wherein a radius of curvature of the central region is between 30 mm and 600 mm.

8. The stopper of any of claims 1-7, wherein each of the first rib and the second rib comprises a radially outward-directed contact surface, and wherein a width of the contact surface is between 0.2 mm and 10 mm and preferably between 0.2 mm and 1.5 mm.

9. The stopper of any of claims 1-8, further comprising a coating applied to at least a radially outward-directed contact surface of the first rib and the second rib, to reduce friction between the first and second ribs and the syringe barrel.

10. The stopper of any of claims 1-9, further comprising a third rib spaced from the second rib, the third rib extending from the outer surface of the main body portion and around the outer circumference of the main body portion, wherein a second inter-rib region is positioned between the second rib and the third rib, with the second inter-rib region comprising a curved inter-rib region where the outer surface of the main body portion is curved radially inward.

11. A syringe, comprising:

   a syringe barrel having a barrel proximal end and a barrel distal end and defining a chamber configured for containing a fluid therein; and
   a plunger assembly axially movable within the chamber of the syringe barrel between a retracted position and an advanced position, the plunger assembly including:

      a plunger having a plunger proximal end and a plunger distal end; and
      the stopper of claim 1, with the stopper formed of a material that is free or substantially free of per- and poly- fluoroalkyl substances (PFAS).

12. The syringe of claim 11, wherein the inter-rib region of the stopper comprises:

   a first abutment region positioned adjacent the first rib;
   a second abutment region positioned adjacent the second rib; and
   a central region positioned between the first and second abutment regions;
   wherein each of the first abutment region and the second abutment region is generally flat, and wherein the central region curves radially inward from the first and second abutment regions.

13. The syringe of claim 12 or claim 13, wherein the stopper contains 0 to 200 parts-per-million of PFAS, and preferably 0 to 50 parts-per-million of PFAS.

14. The syringe of any of claims 11-13, wherein an interference between the first and second ribs and the syringe barrel is in the range of 1.5 to 20%, as determined by:

$$\text{Interference} = ((D_{outer\_ribs} / ID_{barrel}) - 1) \times 100,$$

wherein $D_{outer\_ribs}$ is an outer diameter of the first and second ribs and $ID_{barrel}$ is an inner diameter of the syringe barrel.

48

14

40

10

26

12

38

20

16

18,28

24

30

35

**FIG. 1**

FIG. 2

EP 4 454 683 A1

**FIG. 3**

$D_{max\_inter\_rib}$

$D_{outer\_ribs}$

$D_{min\_inter\_rib}$

FIG. 4

**FIG. 5**

Europäisches
Patentamt

European
Patent Office

Office européen
des brevets

## EUROPEAN SEARCH REPORT

Application Number

EP 23 18 7870

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/031239 A1 (BECTON DICKINSON CO [US]) 15 February 2018 (2018-02-15) * paragraph [0049]; figures 7,5b,6 * * paragraph [0055] * | 1-9, 11-14 | INV. A61M5/315 |
| X | WO 2022/109250 A1 (GORE & ASS [US]) 27 May 2022 (2022-05-27) * paragraph [00077]; figure 7 * * paragraph [00079] * * paragraph [00080] * * paragraph [00082] * | 1-4,6-14 | |
| X | US 7 766 882 B2 (DAIKYO SEIKO LTD [JP]) 3 August 2010 (2010-08-03) * figures 1a,1b,2 * | 1-4,6-14 | |

TECHNICAL FIELDS
SEARCHED        (IPC)

A61M

The present search report has been drawn up for all claims

| Place of search | Date of completion of the search | Examiner |
|---|---|---|
| Munich | 14 June 2024 | Hausmann, Alexander |

EPO FORM 1503 03.82 (P04C01)

## ANNEX TO THE EUROPEAN SEARCH REPORT
## ON EUROPEAN PATENT APPLICATION NO.

EP 23 18 7870

This annex lists the patent family members relating to the patent documents cited in the above-mentioned European search report.
The members are as contained in the European Patent Office EDP file on
The European Patent Office is in no way liable for these particulars which are merely given for the purpose of information.

14-06-2024

| Patent document cited in search report | | Publication date | Patent family member(s) | | Publication date |
|---|---|---|---|---|---|
| WO 2018031239 | A1 | 15-02-2018 | AU 2017308574 | A1 | 21-03-2019 |
| | | | CA 3032298 | A1 | 15-02-2018 |
| | | | CN 109562226 | A | 02-04-2019 |
| | | | EP 3496788 | A1 | 19-06-2019 |
| | | | JP 2019524306 | A | 05-09-2019 |
| | | | US 2018043102 | A1 | 15-02-2018 |
| | | | WO 2018031239 | A1 | 15-02-2018 |
| WO 2022109250 | A1 | 27-05-2022 | AU 2021382676 | A1 | 06-07-2023 |
| | | | CA 3196910 | A1 | 27-05-2022 |
| | | | CN 116457043 | A | 18-07-2023 |
| | | | EP 4247459 | A1 | 27-09-2023 |
| | | | JP 2023550943 | A | 06-12-2023 |
| | | | KR 20230110316 | A | 21-07-2023 |
| | | | US 2024001036 | A1 | 04-01-2024 |
| | | | WO 2022109250 | A1 | 27-05-2022 |
| US 7766882 | B2 | 03-08-2010 | CA 2529150 | A1 | 27-06-2006 |
| | | | DK 1674121 | T3 | 06-05-2013 |
| | | | EP 1674121 | A1 | 28-06-2006 |
| | | | JP 2006181027 | A | 13-07-2006 |
| | | | US 2006178643 | A1 | 10-08-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10751473 B